(19) Europäisches Patentamt / European Patent Office / Office européen des brevets

(11) **EP 2 671 598 A2**

(12) **EUROPEAN PATENT APPLICATION**
published in accordance with Art. 153(4) EPC

(43) Date of publication:
**11.12.2013 Bulletin 2013/50**

(51) Int Cl.:
*A61L 27/54* (2006.01)        *A61L 27/04* (2006.01)
*B23K 26/36* (2006.01)       *A61F 2/30* (2006.01)

(21) Application number: **11855061.5**

(22) Date of filing: **09.11.2011**

(86) International application number:
**PCT/KR2011/008508**

(87) International publication number:
**WO 2012/093772 (12.07.2012 Gazette 2012/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.01.2011 KR 20110000439**

(71) Applicant: **Corentec Co., Ltd.**
**Chungcheongnam-do 331-822 (KR)**

(72) Inventors:
• **SUN, Doo-Hoon**
**Seoul 140-210 (KR)**

• **KIM, Yong-Sik**
**Seoul 137-060 (KR)**
• **KIM, Jung-Sung**
**Cheonan-si**
**Chungcheongnam-do 331-740 (KR)**
• **SHIN, Tae-Jin**
**Cheonan-si**
**Chungcheongnam-do 330-831 (KR)**
• **KIM, Byung-Soo**
**Seoul 143-224 (KR)**

(74) Representative: **Klunker . Schmitt-Nilson . Hirsch**
**Destouchesstraße 68**
**80796 München (DE)**

(54) **IMPLANT FOR IN-VIVO INSERTION WHICH IS FORMED WITH A POROUS COATING LAYER THEREON**

(57)   The present invention relates to an implant which is surgically inserted in vivo such as an artificial knee joint or artificial hip joint. More particularly, the present invention relates to an implant for in-vivo insertion, wherein the porosity of a porous coating layer formed on the surface of the implant, thus increasing the bone adhesion of the implant into pores, the adhesivity between the implant and the porous coating layer and the adhesivity between particles in the porous coating layer, wherein vertically-curved pores each having a radius of 100 - 300 $\mu$m are formed in the porous coating layer to increase the adhesivity of the implant to the bone growing into the pores, thus increasing bone adhesion, and wherein the ratio of interconnected pores in the porous coating layer is increased, and thus bones growing into the pores are interconnected, thereby increasing the adhesivity between the implant and the bones.

【FIG. 3】

## Description

### Technical Field

The present invention relates to an implant which is surgically inserted in vivo such as an artificial knee joint or artificial hip joint. More particularly, the present invention relates to an implant for in-vivo insertion, wherein the porosity of a porous coating layer formed on the surface of the implant, thus increasing the bone adhesion of the implant into pores, the adhesivity between the implant and the porous coating layer and the adhesivity between particles in the porous coating layer, wherein vertically-curved pores each having a radius of 100 - 300 $\mu$m are formed in the porous coating layer to increase the adhesivity of the implant to the bone growing into the pores, thus increasing bone adhesion, and wherein the ratio of interconnected pores in the porous coating layer is increased, and thus bones growing into the pores are interconnected, thereby increasing the adhesivity between the implant and the bones.

### Background Art

Implants for in-vivo insertion are objects inserted into  the human body by a surgical operation. Examples of implants may include: a femur bonding member and a tibia bonding member which are surgically inserted into a femoral region and a tibial region for the purpose of an artificial knee joint surgery; and an acetabular cup and a femoral stem which are surgically inserted into a hip joint region and a femoral region for the purpose of an artificial hip joint surgery.

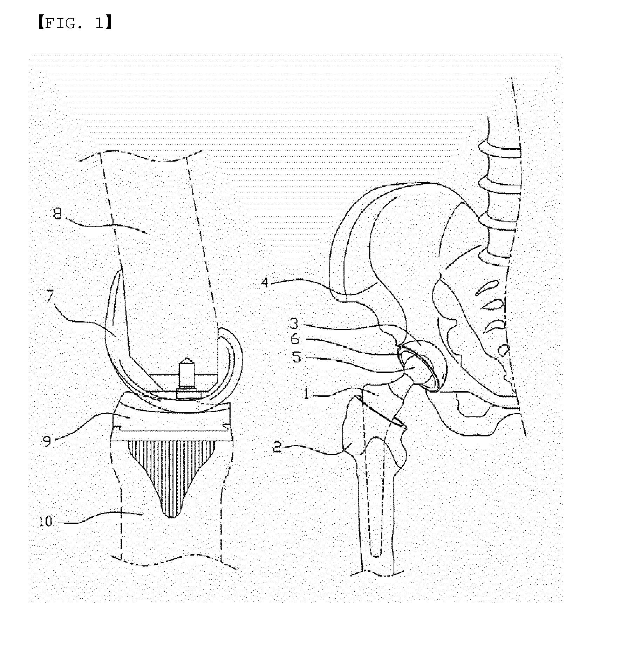

As an example of implants for in-vivo insertion, an artificial hip joint, as shown in FIG. 1, includes an acetabular cup 3 fixed in an acetabulum of the pelvis and a femoral stem 1 inserted and fixed in a femur 2. Each of the femoral stem 1 and the acetabular cup 3 is made of a titanium alloy or the like that is harmless to the human body. The femoral stem 1 is provided at an end thereof with a femur head 5 which is made of ceramic or a metal material, and the acetabular cup 3 is provided therein with a hemispherical seal 6 in which the femur head 5 is disposed and rotated. The hemispherical seal 6 is made of a ceramic material or polyethylene. Such an artificial hip joint is configured such that the femur head 5 can be rotated on the hemispherical seal 6 by the movement of the femur 2 and the femoral stem 1. Further, as an example of implants for in-vivo insertion, an artificial knee joint, as shown in FIG. 1, is configured such that a femur bonding member 7 is fixed at an end of the femur 8 (the end facing the tibia 10), and a tibia bonding member 9  is fixed at an end of the tibia 10 (the end facing the thigh bone 8), and thus the femur bonding member 7 can be rotated on the tibia bonding member 9.

As raw materials of implants such as the femoral stem 1, the acetabular cup 3, the femur bonding member 7 and the tibia bonding member 9, titanium, a titanium alloy, a cobalt-chromium alloy and the like have been generally used. Particularly, among these raw materials, titanium and a titanium alloy are most widely used, because they can be easily processed, and they have excellent biological affinity, mechanical strength and corrosion resistance, and thus they can be suitably used as biomaterials. However, an implant made of only titanium, a titanium alloy or a chromium-cobalt alloy is problematic in that the probability of the implant failing in implantation increases because the initial time taken in bonding the implant with bone is long at the time of implanting the implant into the human body.

In order to solve such a problem, there is proposed a method of forming a porous coating layer on the surface of an implant made of only titanium, a titanium alloy or a chromium-cobalt alloy. However, this method is also problematic in that it is difficult to increase the ratio of pores in the porous coating layer, that is, the porosity of the porous coating layer formed on the surface of the implant (generally, as porosity increases, bone adhesion increases because bone grows into pores), and the adhesion strength between the porous coating layer and the implant (matrix material) and the adhesivity between particles in the porous coating layer become relatively weak when the porosity of the porous coating layer is arbitrarily increased, so that the porous coating layer formed on the surface of the implant is easily detached by friction at the time of implanting the implant, and the detached porous coating layer inhibits the growth of the implant into bone, with the result that stress disintegration effects are reduced, and thus the implant cannot be strongly fixed in the bone.

Further, this method is also problematic in that, although it is required for increase of bone adhesion to interconnect the bones growing into the pores formed in the porous coating layer by interconnecting the pores, that is, by forming passages, it is difficult to form a porous coating layer provided therein with interconnected pores.

Further, this method is also problematic in that, although it is required for increase of bone adhesion to form curved pores in the porous coating layer, it is difficult to form a porous coating layer provided therein with precisely-controlled curved pores.

**Disclosure**

**Technical Problem**

Accordingly, the present invention has been devised to solve the above-mentioned problems, and an object of the present invention is to provide an implant for in-vivo insertion including a porous coating layer formed on the surface thereof, wherein the porosity of a porous coating layer formed on the surface of the implant, thus increasing the bone adhesion of the implant into pores, the adhesivity between the implant and the porous coating layer and the adhesivity between particles in the porous coating layer.

Another object of the present invention is to provide an implant for in-vivo insertion including a porous coating layer formed on the surface thereof, wherein vertically-curved pores each having a radius of 100 - 300 $\mu$m are formed in the porous coating layer to increase the adhesivity of the implant to the bone growing into the pores, thus increasing bone adhesion.

Still another object of the present invention is to provide an implant for in-vivo insertion including a porous coating layer formed on the surface thereof, wherein the ratio of interconnected pores in the porous coating layer is increased, and thus bones growing into the pores are interconnected, thereby increasing the adhesivity between the implant and the bones.

**Technical Solution**

In order to accomplish the above objects, an implant for  in-vivo insertion according to the present invention includes the following constituents.

In an aspect of the present invention, the implant for in-vivo insertion includes: a porous coating layer formed on an outer surface of the implant, wherein the porous coating layer is formed by applying metal powder onto an implant metal using a metal-based rapid prototyping technology, and is formed under the conditions of a tool course and a laser process such that it has a thickness of 200 - 1000 $\mu$m and is provided therein with pores having a size of 150 - 800 $\mu$m at a porosity of 40 - 70 vol%, thus increasing the porosity of the porous coating layer and increasing the adhesivity between the implant and the porous coating layer and the adhesivity between metal powder particles in the porous coating layer.

In the implant, the porous coating layer may include vertically curved pores having a radius of 100 - 300 $\mu$m, thus increasing the adhesivity of the porous coating layer to bone growing into the pores.

Further, in the implant, the porous coating layer may be formed according to a tool course continuously repeated in the direction of right-forward-left-forward to increase the ratio of interconnected pores in the porous coating layer, and thus bones growing into the pores are interconnected, thereby increasing adhesivity between the porous coating layer and the interconnected bones.

Further, in the implant, the implant metal may be a biocompatible material selected from the group consisting of titanium (Ti), a titanium (Ti) alloy, a cobalt-chromium (Co-Cr) alloy and a stainless steel alloy, and the metal powder may be biocompatible material powder selected from the group consisting of titanium (Ti) powder, titanium (Ti) alloy powder and cobalt-chromium (Co-Cr) alloy powder.

**Advantageous Effects**

The implant for in-vivo insertion according to the present invention can exhibit the following effects.

According to the implant of the present invention, the porosity of a porous coating layer formed on the surface of the implant, thus increasing the bone adhesion of the implant into pores, the adhesivity between the implant and the porous coating layer and the adhesivity between particles in the porous coating layer.

According to the implant of the present invention, vertically-curved pores each having a radius of 100 - 300 $\mu$m are formed in the porous coating layer to increase the adhesivity of the implant to the bone growing into the pores, thus increasing bone adhesion.

According to the implant of the present invention, the ratio of interconnected pores in the porous coating layer is increased, and thus bones growing into the pores are  interconnected, thereby increasing the adhesivity between the implant and the bones.

**Description of Drawings**

FIG. 1 is a reference view showing an artificial hip joint and an artificial knee joint as examples of implants.

FIG. 2 is a view explaining a metal-based rapid prototyping technology.

FIG. 3 is a perspective view showing implants (artificial hip joint and artificial knee joint) for in-vivo insertion including a porous coating layer formed thereon according to an embodiment of the present invention.

FIG. 4 is an electron microscope photograph showing the pore size of the porous coating layer of FIG. 3.

FIG. 5 is an electron microscope photograph showing the pore shape and thickness of the porous coating layer of FIG. 3.

FIG. 6 is an electron microscope photograph showing the connection state of pores of the porous coating layer of FIG. 3.

FIG. 7 is a reference view showing an implant whose pore shape and size were adjusted using a tool course.

FIG. 8 is a photograph showing a specimen used in Test 1.

FIG. 9 is a photograph showing a test apparatus used in Test 1.

FIG. 10 is a photograph showing a specimen used in Test 2.

FIG. 11 is a photograph showing a test apparatus used in Test 2.

FIG. 12 is a photograph showing a specimen used in Test 3.

FIG. 13 is a photograph showing a test apparatus used in Test 3.

FIG. 14 is a graph showing the statistical data of shear stress values measured in Test 3.

FIG. 15 is a photograph showing a specimen used in Test 4.

FIG. 16 is a photograph showing a test apparatus used in Test 4.

FIG. 17 is a reference view showing the actuation principle of the test apparatus of FIG. 16.

<Description of the Reference Numerals in the Drawings>

| | |
|---|---|
| a: implant | b: porous coating layer |
| c: pore | 101: specimen |
| 102: laser beam | 103: molten paste |
| 104: cladding material | 105: cladding layer |
| 1: femoral stem | 2: femur |
| 3: acetabular cup | 4: pelvis |
| 5: femur head | 6: hemispherical seal |
| 7: femur bonding member | 8: femur |
| 9: tibia bonding member | 10: tibia |

**Best Mode**

Hereinafter, preferred embodiments of the present invention will be described in detail with reference to the attached drawings.

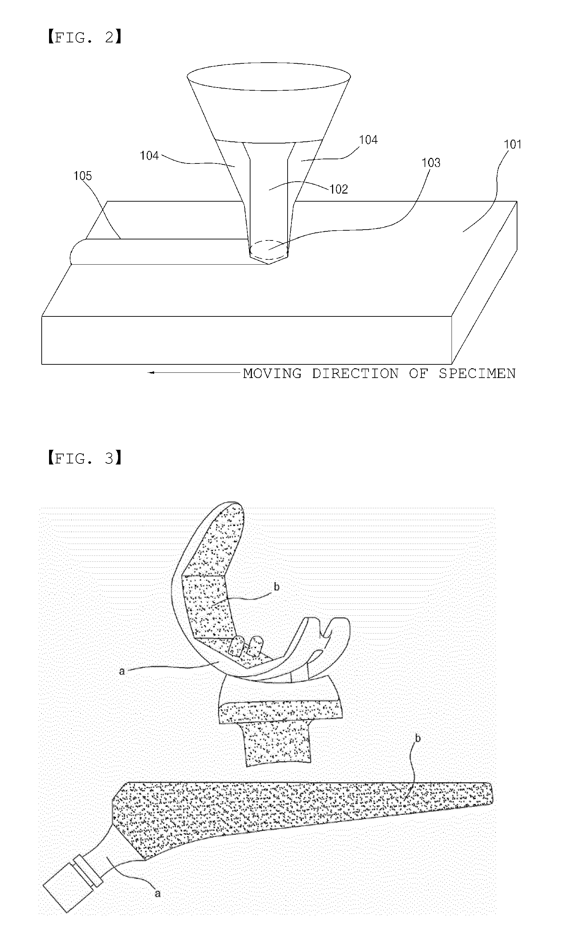

FIG. 2 is a view explaining a metal-based rapid prototyping technology.

Hereinafter, a metal-based rapid prototyping technology, which is a process technology for forming a porous coating layer, will be described, and then an implant for in-vivo insertion, including a porous coating layer formed thereon, according to the present invention will be described.

The metal-based rapid prototyping technology is a new-concept rapid prototyping technology of directly manufacturing a three-dimensional product or manufacturing a tool necessary for the three-dimensional product in a very short period of time using geometric data (three-dimensional CAD data, CT data, MRI data, digital data measured by a three-dimensional data, etc.) stored in a computer. When this metal-based rapid prototyping technology is used, complicated final products and various kinds of tools can be manufactured more rapidly compared to when conventional cutting, casting and the like using CNC (computer numerical control) and other working machines are used. The term "metal-based rapid prototyping technology" used in the present invention is used as a concept including technologies such as SLS (Selective Laser Sintering), DMLS (Direct Metal Laser Sintering), SLM (Selective Laser Melting), EBM (Electron Beam Melting), DMT (laser-aided Direct Metal Tooling), LENS (Laser-Engineered Net Shaping), DMD (Direct Metal Deposition), DMF (Direct Metal Fab) and the like.

In the metal-based rapid prototyping technology, as shown in FIG. 2, the surface of a specimen 101 is irradiated with a laser beam to make molten paste 103 locally, and simultaneously a powdered cladding material 104 (for example, a metal, a metal alloy or the like) is supplied to form a new cladding layer 105 on the surface of the specimen 101. According to the metal-based repaid prototyping technology, two-dimensional section information is computed from three-dimensional CAD data, and cladding layers having shape and thickness and/or height corresponding to the two-dimensional section information are sequentially formed, thus rapidly forming a three-dimensional functional metal product or a tool. In this case, the shape and height of the cladding layer is precisely set by controlling a tool course computed from two-dimensional section information and process variables such as laser output, mode and intensity of laser beam, moving speed of a specimen, characteristics of cladding powder, amount of supply of cladding powder, falling speed of cladding powder and the like. Therefore, in the present invention, the porosity as well as height and pore size and shape of the porous coating layer are obtained using the metal-based rapid prototyping technology, thus increasing bone adhesion and increasing the adhesivity of the femoral stem to bone.

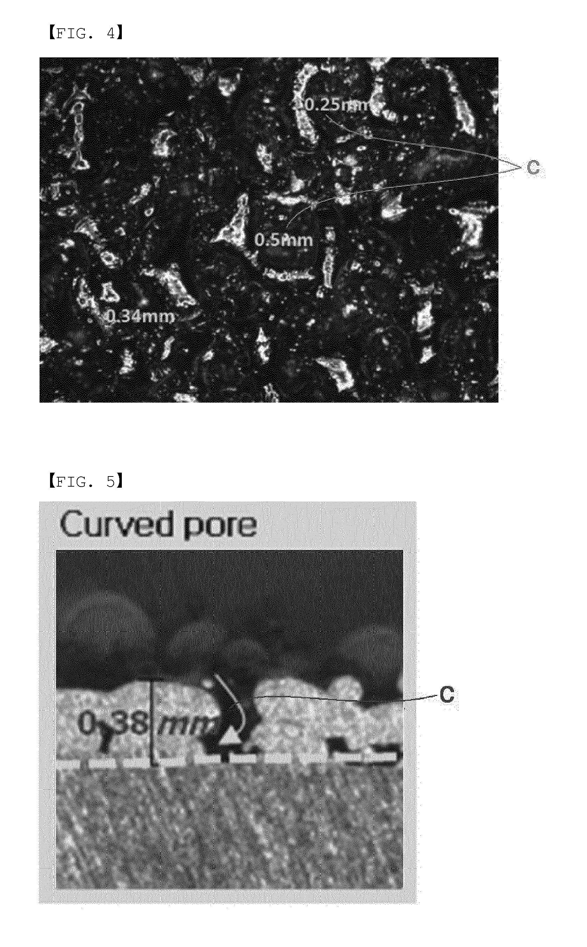

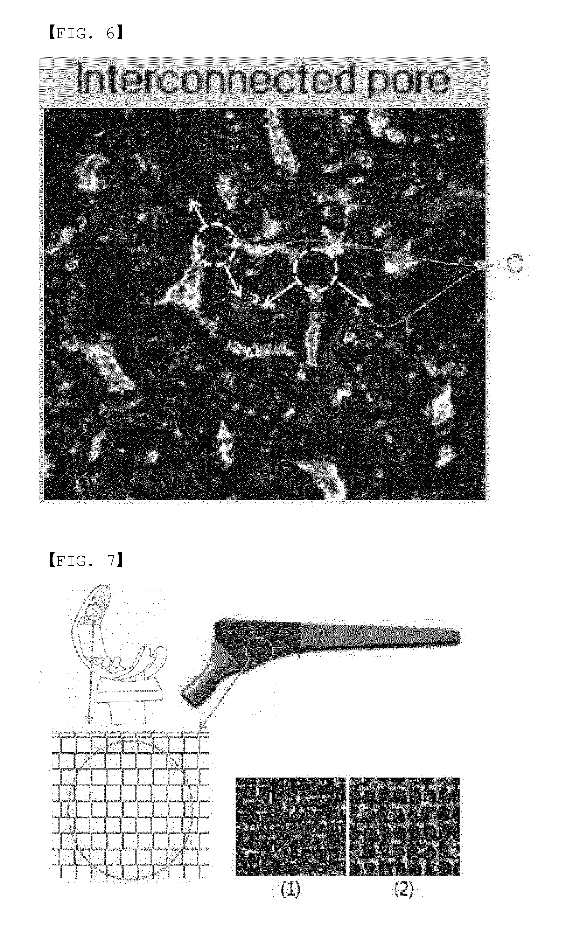

FIG. 3 is a perspective view showing implants (artificial hip joint and artificial knee joint) for in-vivo insertion including a porous coating layer formed thereon according to an embodiment of the present invention, FIG. 4 is an electron microscope photograph showing the pore size of the porous coating layer of FIG. 3, FIG. 5 is an electron microscope photograph showing the pore shape and thickness of the porous coating layer of FIG. 3, FIG. 6 is an electron microscope photograph showing the connection state of pores of the porous coating layer of FIG. 3, and FIG. 7 is a reference view showing an implant whose pore shape and size were adjusted using a tool course.

Referring to FIGS. 3 to 7, an implant for in-vivo insertion according to an embodiment of the present invention (in FIG. 3, an artificial knee joint and a femoral stem for an artificial hip joint are shown in examples of the implant) includes a porous coating layer (b) formed on an outer surface of the implant (a), wherein the porous coating layer (b) is formed by applying metal powder to the surface of the implant (a) using a metal-based rapid prototyping technology, and is formed under the conditions of a tool course and a laser process such that it has a thickness of 200 - 1000 $\mu$m and is provided therein with pores having a size of 150 - 800 $\mu$m at a porosity of 40 - 70 vol%, thus increasing the porosity of the porous coating layer (b) and increasing the adhesivity between the implant (a) and the porous coating layer (b) and the adhesivity between metal powder particles in the porous coating layer (b).

The implant (a) is made of titanium, a titanium alloy, a cobalt-chrome alloy or a stainless steel alloy, which is generally used as a biocompatible material because it has excellent bioaffinity, mechanical strength and corrosion resistance. In order to increase the success rate of transplanting the implant (a) into the human body by decreasing the initial bonding time of the implant (a) and bone, the porous coating layer (b) is formed on the outer surface of the implant (a).

The porous coating layer (b) is configured such that pores are formed on the surface of the implant (a) using biocompatible material powder such as titanium powder, titanium alloy powder, cobalt-chromium alloy powder or the like, thus increasing the adhesivity between the implant (a) and bone using the growth of the implant (a) into the bone at the time of transplanting the implant (a) into the human body. Conventionally, attempts to form a coating layer having pores on the surface of the implant (a) have been conducted. However, these conventional attempts are problematic in that it is difficult to increase the ratio of pores in the porous coating layer, that is, the porosity of the porous coating layer formed on the surface of the implant (generally, as porosity increases, bone adhesion increases because bone grows into pores), and the adhesion strength between the porous coating layer (b) and the implant (a) (matrix material) and the adhesivity between particles in the porous coating layer (b) become relatively weak when the porosity of the porous coating layer (b) is arbitrarily increased, so that the porous coating layer (b) formed on the surface of the implant (a) is easily detached by friction at the time of transplanting the implant (a) into the human body, and the detached porous coating layer (b) inhibits the growth of the implant (a) into bone, with the result that stress disintegration effects are reduced, and thus the implant (a) cannot be strongly fixed in the bone. In the present invention, as described above, the porosity as well as height and pore size and shape of the porous coating layer (b) are obtained using the metal-based rapid prototyping technology, thus increasing bone adhesion and increasing the adhesivity of the femoral stem to bone and the adhesivity between particles in the porous coating layer (b) .

Particularly, the implant of the present invention is characterized in that the porous coating layer (b) is formed to have a thickness of 200 - 1000 $\mu$m (refer to FIG. 5), and is provided therein with pores having a size of 150 - 800 $\mu$m (refer to FIG. 4) at a porosity of 40 - 70 vol% (refer to FIG. 4), thus increasing the porosity of the porous coating layer (b) and increasing the adhesivity between the implant (a) and the porous coating layer (b) and the adhesivity between metal powder particles in the porous coating layer (b). That is, generally, in order to increase only the adhesion of the porous coating layer (b) formed on the surface of the implant (a) to the bone growing into the porous coating layer (b), that is, only the bone adhesion, it is advantageous to increase the porosity of the porous coating layer (b). However, in this case, there is caused a problem in that the adhesion strength between the porous coating layer (b) and the implant (a) (matrix material) and the adhesivity between particles in the porous coating layer (b) become relatively weak, so that the porous coating layer (b) formed on the surface of the implant (a) is easily detached by friction at the time of transplanting the implant (a) into the human body, and the detached porous coating layer (b) inhibits the growth of the implant (a) into bone, with the result that stress disintegration effects are reduced, and thus the implant (a) cannot be strongly fixed in the bone. Therefore, in the present invention, as described above, the thickness of the porous coating layer (b) is maintained at 200 - 1000 $\mu$m, and the size of pores in the porous coating layer (b) is maintained at 150 - 800 $\mu$m, thus obtaining a relatively high porosity of 40 - 70 vol% and maintaining the high adhesion strength between the porous coating layer (b) and the implant (a) (matrix material) and the high adhesivity between particles in the porous coating layer (b) (These facts will be verified by the following test data).

Further, in the present invention, as shown in FIG. 5, the porous coating layer (b) includes vertically curved pores (c) having a radius of 100 - 300 μm, and thus the adhesivity of the porous coating layer (b) to the bone growing into the pores (c) can be increased. That is, pores (c), through which bone grows into the porous coating layer (b), are formed in the shape of vertically curved pores having a radius of 100 ~ 300 μm rather than vertically linear pores, so the bone growing into pores (c) is grown to the lower end of the pores (c), thereby increasing the adhesivity between the bone and the implant (a) compared to the adhesivity between the bone and the porous coating layer (b).

Further, in the present invention, as shown in FIG. 6, the pores (c) formed in the porous coating layer (b) are interconnected, and thus bones growing into the pores are interconnected, thereby increasing bone adhesion. Particularly, as shown in FIG. 7, the porous coating layer (b) is formed according to a tool course continuously repeated in the direction of right-forward-left-forward to increase the ratio of interconnected pores in the porous coating layer (b), and thus the bones growing into the pores are interconnected, thereby relatively increasing bone adhesion. For reference, (1) and (2) of FIG. 7 are electron microscope photographs of the sequentially-magnified pores (c) formed in the porous coating layer (b).

Hereinafter, the fact that the implant (a) including the porous coating layer (b) according to the present invention has a relatively high porosity of 40 - 70 vol% and the fact that the adhesion strength between the implant (a) (matrix material) and the porous coating layer (b) and the adhesivity between metal powder particles in the porous coating layer (b) are also excellent will be verified by test data.

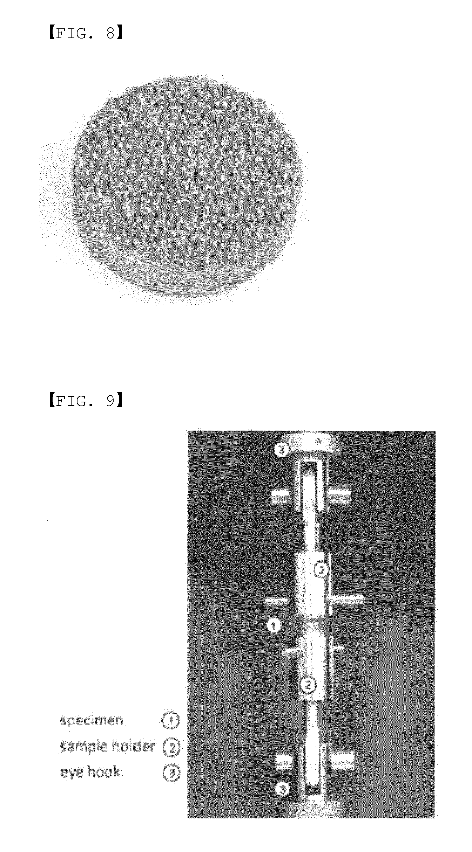

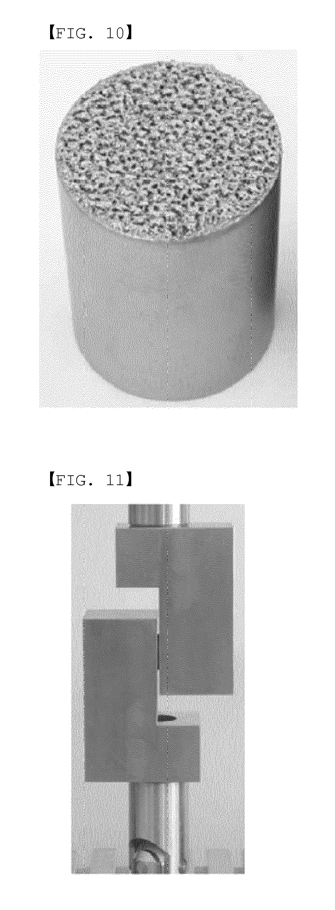

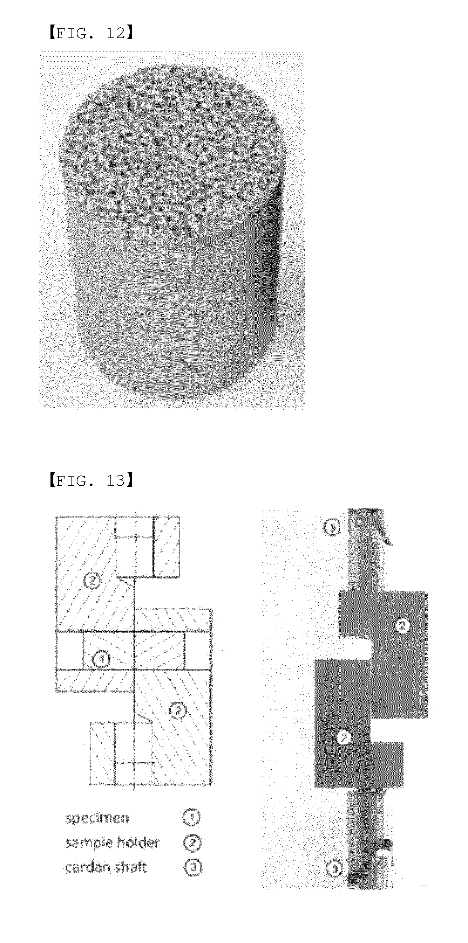

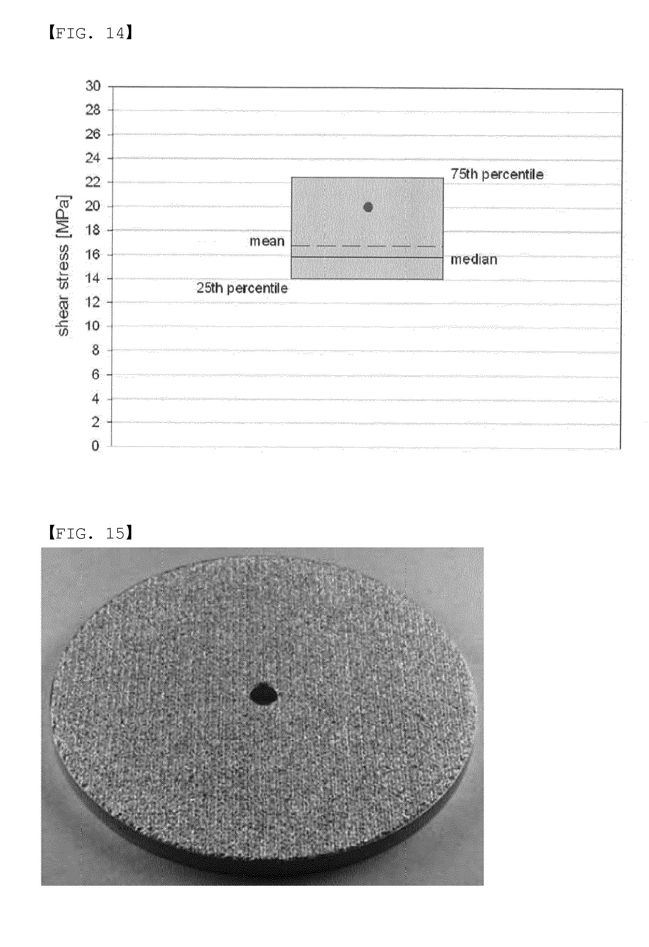

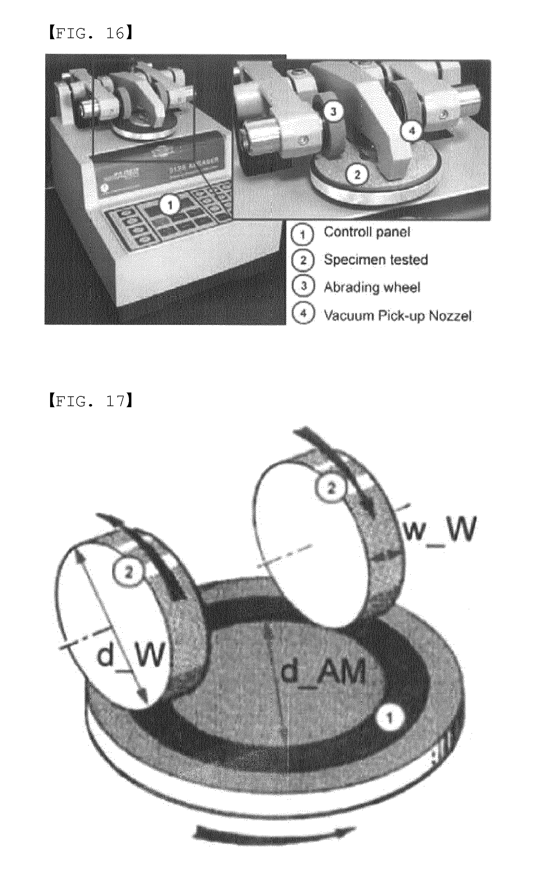

FIG. 8 is a photograph showing a specimen used in Test 1, FIG. 9 is a photograph showing a test apparatus used in Test 1, FIG. 10 is a photograph showing a specimen used in Test 2, FIG. 11 is a photograph showing a test apparatus used in Test 2, FIG. 12 is a photograph showing a specimen used in Test 3, FIG. 13 is a photograph showing a test apparatus used in Test 3, FIG. 14 is a graph showing the statistical data of shear stress values measured in Test 3, FIG. 15 is a photograph showing a specimen used in Test 4, FIG. 16 is a photograph showing a test apparatus used in Test 4, and FIG. 17 is a reference view showing the actuation principle of the test apparatus of FIG. 16.

[Test 1] Test of tensile force of an implant (a) provided with a porous coating layer (b)

- Purpose: measurement of adhesivity or inner cohesion of a coating layer formed on an implant
- Specimen: five specimens of FIG. 8, each of which was prepared by applying a coating layer having a thickness of 200 ~ 1000 μm, a pore size of 150 - 800 μm and a porosity of 40 ~ 70 vol% onto a titanium matrix material having a size of 25.4 mm (diameter) x 6.3 5 mm (height)
- Test standard: ASTM F 1147, which is the standard for testing tensile force of a coating layer by U.S. FDA
- Test method: This test was conducted by placing a specimen between upper and lower sample holders of a tensile force test apparatus (Model No. 360, manufactured by EndoLab Corporation in Germany) shown in FIG. 9 and then applying a tensile load to the specimen at a rate of 2.5 mm/min
- Test result: tensile forces of the specimens calculated by the following Equation are given in Table 1 below:

$$\sigma^{tensile} = F / \{(d/2)^2 * \Pi\}$$

($\sigma^{tensile}$: tensile force, F: applied load, d: size (25.4mm))

[Table 1]

| Specimen | Maximum load (kN) | Maximum tensile strength (MPa) |
|----------|-------------------|-------------------------------|
| 1.1 | 26.89 | 53.07 |
| 1.2 | 25.95 | 51.22 |
| 1.3 | 21.97 | 43.36 |
| 1.4 | 22.65 | 44.71 |
| 1.5 | 25.61 | 50.54 |
| Average | **24.62** | **48.58** |

From the results of Table 1 above, it can be ascertained that the average tensile strength of the implant (a) provided with the porous coating layer (b) is 48.58 MPa, which exceeds 22 MPa (value determined by the test standard), and thus this implant (a) has excellent tensile strength, and that coating layers were not separated from all of the specimens.

[Test 2] Test of constant-volume shear force of an implant (a) provided with a porous coating layer (b)

- Purpose: measurement of adhesivity or inner cohesion of a coating layer formed on an implant
- Specimen: five specimens of FIG. 10, each of which was prepared by applying a coating layer having a thickness of 200 ~ 1000 $\mu$m, a pore size of 150 - 800 $\mu$m and a porosity of 40 ~ 70 vol% onto a titanium matrix material having a size of 19.05 mm (diameter) x 25.4 mm (height)

- Test standard: ASTM F 1044, which is the standard for testing shear force of a coating layer by U.S. FDA

- Test method: This test was conducted by inserting a specimen between left and right sample holders of a shear force test apparatus (Model No. 292, manufactured by EndoLab Corporation in Germany) shown in FIG. 11 and then applying a shear load to the specimen at a rate of 2.5 mm/min
- Test result: shear forces of the specimens calculated by the following Equation are given in Table 2 below:

$$\sigma^{shear} = F / \{(d/2)^2 * \pi\}$$

($\sigma^{shear}$: shear force, F: applied load, d: size (19.05mm))

[Table 2]

| Specimen | Maximum load (kN) | Maximum shear strength (MPa) |
|---|---|---|
| 1.1 | 13.00 | 45.61 |
| 1.2 | 13.06 | 45.81 |
| 1.3 | 14.17 | 49.72 |
| 1.4 | 12.97 | 45.52 |
| 1.5 | 12.84 | 45.05 |
| Average | **13.21** | **46.34** |

From the results of Table 1 above, it can be ascertained that the average shear strength of the implant (a) provided with the porous coating layer (b) is 46.34 MPa, which exceeds 20 MPa (value determined by the test standard), and thus this implant (a) has excellent shear strength, and that coating layers were not separated from all of the specimens.

[Test 3] Test of fatigue shear force of an implant (a) provided with a porous coating layer (b)

- Purpose: measurement of shear fatigue and bending fatigue performances of a coating layer formed on an implant
- Specimen: seven specimens of FIG. 12, each of which was prepared by applying a coating layer having a thickness of 200 ~ 1000 $\mu$m, a pore size of 150 - 800 $\mu$m and a porosity of 40 ~ 70 vol% onto a titanium matrix material having a size of 19.05 mm (diameter) x 25.4 mm (height)
- Test standard: ASTM F 1160, which is the standard for testing shear and bending fatigues of a coating layer by U.S. FDA
- Test method: This test was conducted by inserting a specimen between left and right sample holders of a shear and bending fatigue test apparatus (Model No. 302, manufactured by EndoLab Corporation in Germany) shown in FIG. 13 and then applying a sine-curved dynamic load having a frequency of 20 Hz to the specimen between maximum load and minimum load (minimum load is set to 10% of maximum load) at a cycle (period) of a maximum of ten million
- Test result: shear forces of the specimens calculated by the following Equation are given in Table 3 below:

$$\sigma^{shear} = F / \{(d/2)^2 * \Pi\}$$

($\sigma^{shear}$: shear force, F: applied load, d: size (19.05mm))

[Table 3]

| Specimen | Minimum load (kN) | Maximum load (kN) | Minimum shear force (MPa) | Maximum shear force (MPa) | Cycle | Bone fracture occurrence |
|---|---|---|---|---|---|---|
| 1.1 | 0.85 | 8.51 | 3.00 | 30.00 | 47,944 | ○ |
| 1.2 | 0.78 | 7.80 | 2.75 | 27.50 | 124,956 | ○ |
| 1.3 | 0.71 | 7.08 | 2.50 | 24.98 | 535,939 | ○ |
| 1.4 | 0.64 | 6.38 | 2.25 | 22.50 | 2,298,912 | ○ |
| 1.5 | 0.50 | 4.96 | 1.75 | 17.50 | 10,000,000 | × |
| 1.6 | 0.57 | 5.67 | 2.00 | 20.00 | 10,000,000 | × |
| 1.7 | 0.57 | 5.67 | 2.00 | 20.00 | 10,000,000 | × |

From the results of Table 3 above, it can be ascertained that, even when a dynamic load was applied at a cycle of ten million, the shear strength of the implant (a) provided with the porous coating layer (b) was maintained at 20.00 MPa, bone fracture did not occur, and a coating layer was not detached. Further, as shown in FIG. 14, it can be ascertained that the values of shear stress obtained in Test 3 exceed the average value thereof on statistical data.

[Test 4] Test of wear resistance of an implant (a) provided with a porous coating layer (b)

- Purpose: measurement of wear resistance of a coating layer formed on an implant
- Specimen: six specimens of FIG. 15, each of which was prepared by applying a coating layer having a thickness of 200 ~ 1000 $\mu$m, a pore size of 150 - 800 $\mu$m and a porosity of 40 ~ 70 vol% onto a titanium matrix material having a size of 100 mm (diameter) x 6 mm (height)
- Test standard: ASTM F 1978, which is the standard for testing wear resistance of a coating layer by U.S. FDA
- Test method: This test was conducted using a wear resistance test apparatus (Model No. 140, 366, manufactured by EndoLab Corporation in Germany) shown in FIG. 16. As shown in FIG. 17, two abrading wheels rotated in a direction opposite to each other by the rotation of a disk on which a specimen is disposed comes into contact with the specimen disposed on the disk, and, at this time, the degree of the specimen being worn is measured. Specifically, test method is conducted by the following steps of: ① measuring the initial weight of a specimen before the test; ② cleaning the specimen and then disposing the cleaned specimen on a disk of the wear resistance test apparatus; ③ bringing the specimen disposed on the disk into contact with two abrading wheels to abrade the specimen; ④ ultrasonically cleaning the abraded specimen for 30 minutes, drying the ultrasonically-cleaned specimen in an oven at 100°C for 10 minutes, and then cooling the dried specimen at room temperature; and ⑤ measuring the weight of this specimen three times. These steps of ② to ⑤ are cumulatively performed at a cycle of 5, 10 and 100.
- Test result: weight losses per cycle of the specimens calculated by the following Equation are given in Table 3 below:

$$dw_n = w_0 - w_n$$

($dw_n$: accumulated weight loss, $w_0$: weight measured during the first three times, $w_n$: average weight measured during three times, n: accumulated cycle number)

[Table 4]

| Specimen | 2.1 | 2.2 | 2.3 | 2.4 | 2.5 | 2.6 | Average |
|---|---|---|---|---|---|---|---|
| Cumulative cycle | weight loss (mg) | weight loss (mg) | weight loss (mg) | weight loss (mg) | weight loss (mg) | weight loss (mg) | weight loss (mg) |
| 0 | 0.00 | 0.00 | 0.00 | 0.00 | 0.00 | 0.00 | 0.00 |
| 2 | 0.50 | 4.20 | 7.30 | 7.00 | 4.40 | 7.00 | 5.07 |
| 5 | 2.20 | 8.60 | 15.10 | 12.00 | 7.50 | 14.50 | 9.98 |
| 10 | 3.70 | 10.70 | 20.60 | 17.00 | 9.60 | 19.90 | 13.58 |
| 100 | 27.80 | 34.00 | 54.60 | 44.80 | 32.60 | 49.60 | 40.57 |

From the result of measuring the weight losses of specimens at an accumulated cycle of 100 times, it is recorded that specimen 2.3 shows a maximum weight loss of 54.6 mg, specimen 2.1 shows a minimum weight loss of 27.80 mg, and average weight loss of specimens is 40.57 mg. The average weight loss thereof (40.57 mg) sufficiently satisfies the average weight loss of 65 mg or less at the time of testing wear resistance at an accumulated cycle of 100 times, defined by FDA. Therefore, it can be ascertained that adhesivity between powder particles in the coating layer of the present invention is also increased.

Considering all the test results, as ascertained from the above test results, when the thickness and pore size and shape of the porous coating layer (b) of the implant (a) of the present invention are accurately controlled, the porous coating layer (b) has a relatively high porosity of 40 - 70 vol%, and simultaneously the adhesion strength between the porous coating layer (b) and the implant (a) (matrix material) and the adhesivity between powder particles in the porous coating layer (b) can be maintained high, so the adhesivity between the implant (b) and the bone growing into the pores (c) of the porous coating layer (b) increases, and the separation of the porous coating layer (b) from the implant (b) can be prevented in the procedure of operating a femoral stem to prevent the retardation of bone growth, the reduction of stress dissipation effects and the looseness of the implant (a) inserted in the human body, thereby preventing the failure of operation of the implant (a).

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. An implant for in-vivo insertion, comprising:

   a porous coating layer formed on an outer surface of the implant,
   wherein the porous coating layer is formed by applying metal powder onto an implant metal using a metal-based rapid prototyping technology, and is formed under the conditions of a tool course and a laser process such that it has a thickness of 200 - 1000 $\mu$m and is provided therein with pores having a size of 150 - 800 $\mu$m at a porosity of 40 - 70 vol%, thus increasing the porosity of the porous coating layer and increasing the adhesivity between the implant and the porous coating layer and the adhesivity between metal powder particles in the porous coating layer.

2. The implant of claim 1, wherein the porous coating layer includes vertically curved pores having a radius of 100 - 300 $\mu$m, thus increasing adhesivity of the porous coating layer to bone growing into the pores.

3. The implant of claim 2, wherein the porous coating layer is formed according to a tool course continuously repeated in the direction of right-forward-left-forward to increase the ratio of interconnected pores in the porous coating layer, and thus bones growing into the pores are interconnected, thereby increasing adhesivity between the porous coating layer and the interconnected bones.

4. The implant of claim 3, wherein the implant metal is a biocompatible material selected from the group consisting of titanium (Ti), a titanium (Ti) alloy, a cobalt-chromium (Co-Cr) alloy and a stainless steel alloy, and the metal powder is biocompatible material powder selected from the group consisting of titanium (Ti) powder, titanium (Ti) alloy powder and cobalt-chromium (Co-Cr) alloy powder.

【FIG. 1】

【FIG. 2】

MOVING DIRECTION OF SPECIMEN

【FIG. 3】

【FIG. 4】

【FIG. 5】

【FIG. 6】

【FIG. 7】

【FIG. 8】

【FIG. 9】

specimen ①
sample holder ②
eye hook ③

【FIG. 10】

【FIG. 11】

【FIG. 12】

【FIG. 13】

specimen        ①
sample holder   ②
cardan shaft    ③

【FIG. 14】

【FIG. 15】

【FIG. 16】

1. Controll panel
2. Specimen tested
3. Abrading wheel
4. Vacuum Pick-up Nozzel

【FIG. 17】